# EUROPEAN PATENT SPECIFICATION

(11) **EP 3 297 542 B1**
(45) Date of publication and mention of the grant of the patent: **29.07.2020**
(21) Application number: 16723137.2
(22) Date of filing: 18.05.2016
(51) Int. Cl.: A61B 17/04, A61F 6/14, A61F 6/18

(54) **DUAL ANCHORING DEVICE FOR THE SUSPENSION OF INTRAUTERINE DEVICES**
DUALES VERANKERUNGSSYSTEM ZUR BEFESTIGUNG INTRAUTERALER VORRICHTUNGEN
SYSTEME DE DOUBLE-ANCRAGE POUR DISPOSITIFS INTRA-UTERINS

(30) Priority: 20.05.2015 EP 15168539
(43) Date of publication of application: 28.03.2018
(73) Proprietor: PAT&Co bvba, 8810 Lichtervelde (BE)
(72) Inventor: WILDEMEERSCH, Dirk, 9000 Ghent (BE)
(74) Representative: Brantsandpatents bvba
(86) International application number: PCT/EP2016/061128
(87) International publication number: WO 2016/184907

(56) References cited:
- WO-A2-2012/088496
- US-A- 4 708 134
- US-A1- 2012 318 276
- D A Grimes ET AL: "Immediate post-partum insertion of intrauterine devices (Review)", , 31 December 2010 (2010-12-31), XP055288894, The Cochrane Collaboration. Retrieved from the Internet: URL:http://onlinelibrary.wiley.com/doi/10. 1002/14651858.CD003036.pub2/epdf [retrieved on 2016-07-15]
- Vandana Dhama ET AL: "Three techniques for skin closure in caesarean section (stapler, absorbable subcuticular, non-absorbable subcuticular suture)", Indian Journal of Obstetrics and Gynecology Research, vol. 3, no. 1, 1 January 2016 (2016-01-01) , page 68, XP055661206, ISSN: 2394-2746, DOI: 10.5958/2394-2754.2016.00014.X

## Description

### FIELD AND BACKGROUND OF THE INVENTION

Immediate postpartum insertion (within 10 minutes of delivery of the placenta) of an intrauterine device or system (IUD/IUS) is generally safe and effective. Studies have confirmed that postpartum IUD insertions, including those done immediately after placental delivery or cesarean section, do not result in increased morbidity. Compared with interval insertions, postpartum insertions do not increase the risk of infection, bleeding, uterine perforation or endometritis, nor do they affect the return of the uterus to its normal size.

Both copper and hormone-releasing IUD/IUS do not affect breastfeeding either, in contrast with systemic hormonal methods. Therefore, the IUD/IUS has a distinct advantage. The post-delivery period may also be a convenient time to have an IUD or an IUS inserted, since it may be one of the few times the woman is in contact with medical services.

In addition, IUDs do not require regular user compliance. Coital-dependent methods may be used inconsistently during the postpartum period by couples who think conception is less likely during this period. Also, if a woman says she wants no more children but has not had time to consider sterilization carefully, an IUD/IUS offers a reversible alternative.

The timing of insertion is important primarily because it influences the risk of expulsion. Expulsion can leave a woman unprotected from pregnancy without her realizing it. Ideally, postpartum insertion should take place within 10 minutes of placental delivery (immediate postplacental) or at about six weeks after birth, when a woman returns for a routine postpartum care visit. For women with limited access to medical care, the time of delivery offers a unique opportunity to address their need for contraception if the delivery takes place in a health-care center. The a priori hypothesis is that this practice is safe, but is associated with a higher expulsion rate than with interval insertion of the IUD.

As the main problem with postpartum insertions is the high expulsion rates of the IUD/IUS seen at that time which is much higher compared to that of interval insertions. The risk of expulsion is lower for insertions done within 10 minutes of delivery than for those done between 10 minutes and hospital discharge. The risk of expulsion can be as much as 40 or 50% which is unacceptably high. For this reason, the method never became popular and general use did not occur. In an attempt to reduce this risk, appropriate training in postpartum insertion techniques has been advocated but with minimum result. It appears, therefore, that the only solution to minimize the number of expulsions is to invent a means for attaching the IUD to the fundus of the uterus immediately post-partum.

Attachment of an IUD to the uterine fundus has been attempted using different techniques and has been the subject of a prior submission (PCT WO91/00714). The anchoring system described in this patent was highly effective and reduced the expulsion rate substantially. However, it was difficult to remove the IUD, especially if required during the first few weeks or even months after insertion. The challenge remains therefore to develop and anchoring means which is easy to insert and remove. US4708134 A discloses an intrauterine contraceptive device (IUD) wherein the fixing device to the fundal part of the uterine cavity is a thread, affixed to the assembly of hollow members, provided with a retaining device in the uterine tissue, adapted for insertion by means of a needle. The thread is secured to a stop means, and is provided at the other end of the passage with a loop allowing cooperation with a split needle. The loop is provided with a knot constituting retaining means in the uterine tissue.

US2012318276 A1 relates to a "frameless" copper-releasing or hormone-releasing intrauterine contraceptive device or system (IUD/IUS). To reduce the risk of expulsion in cases where the uterine wall is soft (e.g., postpartum) biodegradable material is added to the anchoring knot. This can be realized by putting a cap on the assembled anchoring knot. The cap covers preferably the upper part of the knot and leaves a loop extending therefrom for permitting the insertion process with an inserter. The biodegradable cap will degrade in weeks or a few months, leaving only the anchoring knot for retention of the contraceptive device in the uterine cavity at the time the uterus has involuted. Exemplary biodegradable material is a polylactide or polycaprolactone.

### SUMMARY OF THE INVENTION

The present invention consequently is directed to an anchoring device, for its insertion and suspension of a copper- releasing device, or a hormone-releasing intrauterine system which can be inserted easily and safely, under direct vision, immediately after removal of the placenta and contraction of the uterus, and which can be removed within days after its insertion in case of a medical indication or urgency. This purpose is achieved by designing a suitable anchor which, when assembled together with the IUD or IUS, can be inserted and fixed quickly in the fundus of the uterus with a specially designed inserter as described previously.

There are several possible modes of realization. In this respect, we refer to previous submissions (US 4,708.134 and US 5,433,218). These patents describe the fixation of frameless copper and hormone-releasing IUDs in the uterus. This mode of realization is also possible for the suspension of active substances in the post-Cesarean section uterus. However, if used as such, the risk of expulsion of the IUD/IUS will still be unacceptably high because of the soft tissue of the wall of the uterus which is not compact enough to retain the small anchor.

Retention of the anchor in the soft tissue is solved by adding material, in the form of biodegradable suture material to secure the position of the anchor in the soft tissue of the uterine fundus by temporarily attaching the anchor to the more resistant and dense tissue of the serosal layer of the uterus which covers the outside of the uterus. As the uterus involutes over weeks and regains its normal size, and the uterine tissue becomes denser, the permanent anchor will be sufficient to retain the IUD/IUS in situ while the temporary attachment suture degrades.

This aim is achieved by using the same anchoring principle as described previously but, in addition, by connecting the anchor to the serosa using a biodegradable suture. In case the permanent anchor consists of a surgical knot, a thin biodegradable suture could be attached to the knot and stitched to the serosa.

This characteristic of the invention is achieved by puncturing the uterus with an applicator immediately after removal of the placenta so that the anchor is visible at the surface of the uterus. The next step is to remove the applicator and to thread the noose of the anchoring knot with a biodegradable suture and fixing it to the serosa.

According to another characteristic of the invention, the anchor is a surgical knot, sufficiently small in diameter to allow easy removal of the attached device when necessary, even early after its insertion.

According to another characteristic of the invention, the anchor is a surgical knot with a small noose to allow threading with a biodegradable suture.

According to another characteristic of the invention, the biodegradable suture degrades completely within a few weeks with significant reduced tensile strength after only a few days or one week.

According to another characteristic of the invention, the biodegradable suture is adapted to be fixed to the serosal layer of the uterus by stitching it to the serosa.

According to another characteristic of the invention, the biodegradable suture is adapted to be fixed to the serosal layer of the uterus by attaching it with a metal or biodegradable clip.

According to yet another characteristic of the invention, IUD/IUS can be frameless or framed.

According to another characteristic of the invention, the hormone-releasing IUS consists of a rod or hollow tube with tail and anchoring means.

### DESCRIPTION OF THE DRAWINGS

The invention will be better understood with reference to the description as well as the attached drawings, which show, purely by way of example, various embodiments of the invention and in which:
FIG. 1 is a frameless IUD of which the anchor consists of a knot, which is inserted though the wall of the post-partum uterus, and which has a noose through which a biodegradable suture is threaded.
FIG. 2 and FIG. 3 show the fixation of the anchoring knot to the serosa of the uterus by stitching the suture to it or by fixing it to the serosa with a metal or biodegradable clip.
FIG. 4 is a representation of a hormone-releasing system with a fixation means attached to the top of the drug delivery system and with removal tail attached to the bottom of it.
FIG. 5 and FIG. 6 is a more detailed representation of the mode of realization to attach the anchoring means and the removal tail to the drug delivery rod described in FIG.4.
FIG. 7 is a representation of a tube-shaped drug delivery compartment to which the anchoring means and removal tail, being a suture with anchoring knot on the top of the suture, is threaded through the lumen of the drug delivery tube and of which the tube is prevented from sliding off the anchoring suture my means of a metal or plastic clips.

### DESCRIPTION OF THE PREFERRED MODE OF REALIZATION

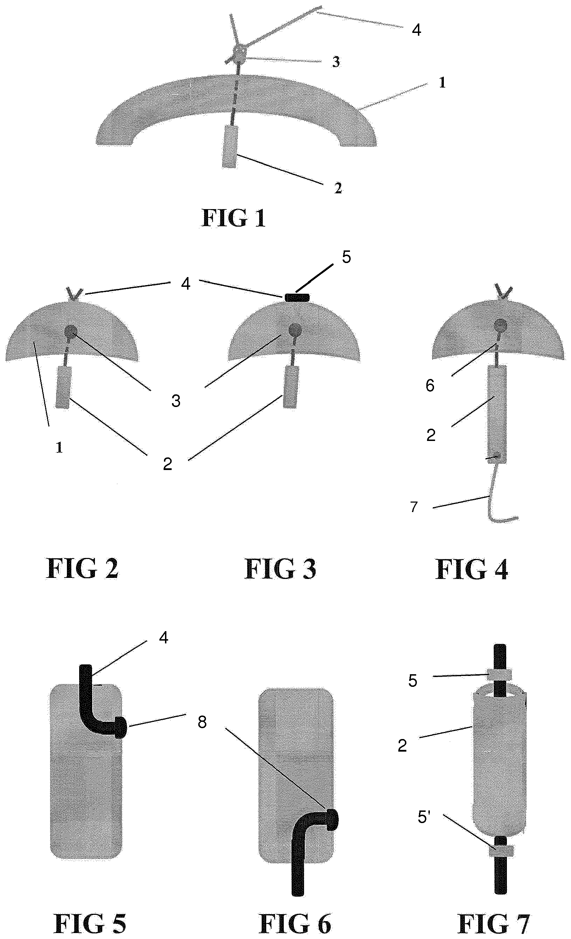

Referring to FIG. 1, showing the fundal part of the uterus 1 with IUD 2 and device anchor 3 which is inserted through the fundal wall and of which the biodegradable suture 4 is inserted through the noose of the anchoring knot 3.

FIG. 2 and FIG. 3 show a segment of the uterine fundus 1 with anchoring knot 3 and the preferred mode of realization consisting of temporarily attaching the anchoring knot to the serosa of the uterus; in Fig. 2 by tying the suture 4 to the serosa and in Fig. 3 by fixing the suture to the serosa with clip 5.

Referring now particularly to FIG. 4, an anchoring means 6 is integrated into the upper part of a drug delivery system 2 for its retention in the uterus, and is provided with a tail 7 also integrated into the drug delivery system for removal of the IUS.

FIG. 5 shows the preferred mode of realization of the attachment of the anchoring suture 4 to the drug delivery rod by spherical thermo-deformation of the end of the suture 8.

FIG. 6 shows a similar mode of realization of the attachment of the removal tail to the drug delivery by spherical thermos-deformation of the end of the tail.

FIG. 7 is a schematic representation of the fixation of a tube-shaped drug delivery system 2 by means of two metal clips 5 and 5' onto the suture which keep the system affixed to the anchoring thread.

The invention has been described and illustrated merely by way of example which is in no way restrictive. Numerous changes in its conception may be made without departing from the invention.

## Claims

1. A drug delivery compartment, suitable for anchoring of an intrauterine device (IUD) or intrauterine system (IUS) (2) to the fundus (1) of a post-Cesarean section uterus, which drug delivery compartment comprises an IUD or IUS, and an anchoring knot (3), whereby said knot (3) comprises a body and a noose, **characterized in that** said drug delivery compartment further comprises a biodegradable suture (4), and whereby threading of the biodegradable suture (4) through said noose allows for a temporary fixation of the IUD or IUS (2) to soft muscular tissue, as to prevent the IUD or IUS (2) from being expelled from the uterus.

2. The drug delivery compartment according to claim 1, **characterized in that** said anchoring knot (3) is threaded with said biodegradable suture (4), and of which the biodegradable suture (4) is suitable to be tied to the serosa of the uterus.

3. The drug delivery compartment according to claim 1, **characterized in that** it further comprises a surgical metal or biodegradable clip (5), configured for fixing said biodegradable suture (4) to the serosa of the uterus.

4. The drug delivery compartment according to any one of claims 1 to 3, **characterized in that** said drug delivery compartment is at the bottom provided with a tail (7) that enables easy removal of said IUD or IUS from the uterine cavity.

5. The drug delivery compartment according to claim 4, **characterized in that** the IUD or IUS comprises a drug delivery rod (2), whereby the anchoring knot (3) and the removal tail (7) are integrated in the top and the bottom of the drug delivery rod (2), by a suture threaded through a preformed curved channel in the rod (2), and fixed in place by a thermoformed spherical thickening at the end of the suture.

6. The drug delivery compartment according to claim 4, **characterized in that** the IUD or IUS (2) is hollow, tube-shaped, and it is fixed onto a suture thread, whereby said tube-shaped IUD or IUS is kept in place by one or more metal or plastic clips (5, 5') on said suture thread, of which at least one clip (5, 5') is placed below said IUD or IUS (2) to keep it affixed to the suture thread.

## Patentansprüche

1. Arzneimittelabgabekammer, geeignet zum Verankern einer intrauterinen Vorrichtung (IUD) oder eines intrauterinen Systems (IUS) (2) am Fundus (1) eines Uterus nach einem Kaiserschnitt, wobei die Arzneimittelabgabekammer eine IUD oder ein IUS und einen Verankerungsknoten (3) umfasst, wobei der Knoten (3) einen Hauptteil und eine Schlaufe umfasst,
**dadurch gekennzeichnet, dass** die Arzneimittelabgabekammer ferner einen biologisch abbaubaren chirurgischen Faden (4) umfasst und wobei das Fädeln des biologisch abbaubaren chirurgischen Fadens (4) durch die Schlaufe eine zeitweise Fixierung der IUD oder des IUS (2) an weichem Muskelgewebe ermöglicht, so dass verhindert wird, dass die IUD oder das IUS (2) vom Uterus ausgestoßen wird.

2. Arzneimittelabgabekammer nach Anspruch 1, **dadurch gekennzeichnet, dass** der Verankerungsknoten (3) mit dem biologisch abbaubaren chirurgischen Faden (4) gefädelt wird und wobei der biologisch abbaubare chirurgische Faden (4) geeignet ist, an die Serosa des Uterus angebunden zu werden.

3. Arzneimittelabgabekammer nach Anspruch 1, **dadurch gekennzeichnet, dass** sie ferner eine chirurgische Metall- oder biologisch abbaubare Klammer (5) umfasst, die dafür gestaltet ist, den biologisch abbaubaren chirurgischen Faden (4) an der Serosa des Uterus zu fixieren.

4. Arzneimittelabgabekammer nach einem der Ansprüche 1 bis 3, **dadurch gekennzeichnet, dass** die Arzneimittelabgabekammer an der Unterseite mit einem Band (7) versehen ist, welches das leichte Entfernen der IUD oder des IUS aus der Gebärmutterhöhle ermöglicht.

5. Arzneimittelabgabekammer nach Anspruch 4, **dadurch gekennzeichnet, dass** die IUD oder das IUS einen Arzneimittelabgabestab (2) umfasst, wobei der Verankerungsknoten (3) und das Entfernungsband (7) durch einen chirurgischen Faden, der durch einen vorgeformten gekrümmten Kanal in dem Stab (2) gefädelt ist, in die Ober- und die Unterseite des Arzneimittelabgabestabes (2) integriert und durch eine warmgeformte kugelförmige Verdickung am Ende des chirurgischen Fadens an Ort und Stelle fixiert sind.

6. Arzneimittelabgabekammer nach Anspruch 4, **dadurch gekennzeichnet, dass** die IUD oder das IUS (2) hohl, röhrenförmig und an einem chirurgischen Faden fixiert ist, wobei die röhrenförmige IUD oder das röhrenförmige IUS durch eine oder mehrere Metall- oder Kunststoffklammern (5, 5') an dem chirurgischen Faden an Ort und Stelle gehalten wird, wobei mindestens eine Klammer (5, 5') unter der IUD oder dem IUS (2) platziert ist, um sie an dem chirurgischen Faden befestigt zu halten.

## Revendications

1. Compartiment de délivrance de médicament, approprié pour l'ancrage d'un dispositif intra-utérin (IUD) ou d'un système intra-utérin (IUS) (2) au fond (1) d'un utérus post-césarienne, lequel compartiment de délivrance de médicament comprend un IUD ou un IUS, et un nœud d'ancrage (3), moyennant quoi ledit nœud (3) comprend un corps et un nœud coulant, **caractérisé en ce que** ledit compartiment de délivrance de médicament comprend en outre un fil de suture biodégradable (4), et moyennant quoi le passage du fil de suture biodégradable (4) à travers ledit nœud coulant permet une fixation temporaire de l'IUD ou de l'IUS (2) à un tissu musculaire souple, de manière à empêcher l'IUD ou l'IUS (2) d'être expulsé de l'utérus.

2. Compartiment de délivrance de médicament selon la revendication 1, **caractérisé en ce que** ledit nœud d'ancrage (3) est traversé par ledit fil de suture biodégradable (4), et dont le fil de suture biodégradable (4) est apte à être lié à la séreuse de l'utérus.

3. Compartiment de délivrance médicament selon la revendication 1, **caractérisé en ce qu'**il comprend en outre un clip chirurgical métallique ou biodégradable (5), configuré pour fixer ledit fil de suture biodégradable (4) sur la séreuse de l'utérus.

4. Compartiment de délivrance de médicament selon l'une quelconque des revendications 1 à 3, **caractérisé en ce que** ledit compartiment de délivrance de médicament est pourvu, en bas, d'une queue (7) qui permet un retrait facile dudit IUD ou IUS de la cavité utérine.

5. Compartiment de délivrance de médicament selon la revendication 4, **caractérisé en ce que** l'IUD ou l'IUS comprend une tige de délivrance de médicament (2), moyennant quoi le nœud d'ancrage (3) et la queue de retrait (7) sont intégrés dans le haut et le bas de la tige de délivrance de médicament (2), par un fil de suture enfilé à travers un canal courbe préformé dans la tige (2), et maintenus en place par un épaississement sphérique thermoformé au niveau de l'extrémité du fil de suture.

6. Compartiment de délivrance de médicament selon la revendication 4, **caractérisé en ce que** l'IUD ou l'IUS (2) est creux, en forme de tube, et il est fixé sur un fil de suture, moyennant quoi ledit IUD ou IUS en forme de tube est maintenu en place par un ou plusieurs clips en métal ou en plastique (5, 5') sur ledit fil de suture, dont au moins un clip (5, 5') est placé au-dessous dudit IUD ou IUS (2) pour le maintenir fixé au fil de suture.
